# EUROPEAN PATENT SPECIFICATION

(11) **EP 2 590 714 B1**
(45) Date of publication and mention of the grant of the patent: **08.03.2017**
(21) Application number: 11730030.1
(22) Date of filing: 08.07.2011
(51) Int. Cl.: A61N 5/06, A61K 9/70, A61K 41/00, A61K 47/36

(54) **Devices containing dry compositions for use in photodynamic therapy**
Vorrichtung mit trockenen Zusammensetzungen für fotodynamische Therapie
Dispositif contenant des compositions sèches destinés à être utilisés dans la thérapie photodynamique

(30) Priority: 09.07.2010 EP 10251232
(43) Date of publication of application: 15.05.2013
(73) Proprietor: Photocure ASA, 0275 Oslo (NO)
(72) Inventor: KLEM, Bjørn, 0286 Oslo (NO); LANGE, Norbert, 1260 Nyon (CH); Groseth, Morten, 0771 Oslo (NO)
(74) Representative: Golding, Louise Ann
(86) International application number: PCT/EP2011/061688
(87) International publication number: WO 2012/004399

(56) References cited:
- WO-A1-96/23543
- WO-A1-2010/026422
- WO-A2-02/09690
- WO-A2-2010/072419
- US-A1- 2001 047 195
- US-A1- 2006 018 956
- US-A1- 2007 260 231
- US-A1- 2009 198 173

## Description

This invention relates to an irradiation device which comprises dry pharmaceutical compositions comprising an active ingredient which is 5-aminolevulinic acid (5-ALA) or a precursor or derivative of 5-ALA or pharmaceutically acceptable salts thereof in the photodynamic treatment (PDT) of cancer, pre-cancerous conditions and non-cancerous conditions of the cervix.

Photodynamic treatment (PDT) is a relatively new technique for the treatment of pre-cancerous lesions, cancer and non-cancerous diseases. PDT involves the administration of a photosensitiser or a precursor thereof to an area of interest. The photosensitiser or precursor thereof is taken up into the cells, where a precursor of a photosensitiser is converted into a photosensitiser. Upon exposure of the area of interest to light, the photosensitiser is excited, usually from a ground singlet state to an excited singlet state. It then undergoes intersystem crossing to a longer-lived excited triplet state. One of the few chemical species present in tissue with a ground triplet state is molecular oxygen. When the photosensitiser and an oxygen molecule are in proximity, an energy transfer can take place that allows the photosensitiser to relax to its ground singlet state, and create an excited singlet state oxygen molecule. Singlet oxygen is a very aggressive chemical species and will very rapidly react with any nearby biomolecules. Ultimately, these destructive reactions will kill cells through apoptosis or necrosis, whereby for instance cancer cells are selectively killed. The mechanisms are still not fully understood, but studies suggest that the clinical result, e.g. the selectivity for cancerous cells, is not due to selective uptake by cancerous cells. Rather, there are similar levels of uptake in all cell types, but the processes of conversion and elimination are different in malignant cells and generally in metabolically active cells, such as inflamed or infected cells, leading to a concentration gradient between cancerous and normal tissue.

Several photosensitisers are known and described in the literature including 5-aminolevulinic acid (5-ALA) and certain derivatives thereof, e.g. 5-ALA esters. 5-ALA and 5-ALA esters are precursors of photosensitisers which are converted to photosensitisers, i.e. protoporphyrins, such as protoporphyrin IX (PpIX). Currently several pharmaceutical products comprising 5-ALA or an ester thereof are in clinical use for PDT. One of them is Metvix^{®}, a dermal product in the form of a cream comprising 5-ALA methyl ester (Galderma, Switzerland), for the photodynamic treatment of actinic keratosis and basal cell carcinoma. Another one is Levulan Kerastick^{®} (DUSA Pharmaceuticals, Canada), a product for the photodynamic treatment of actinic keratosis which contains 5-ALA.

One of the most serious infections of the cervix is an infection with human papilloma virus (HPV) which can develop into cervical cancer. HPV infection is a common factor in the development of almost all cervical cancer cases. Estimates for the prevalence of HPV infections vary, but can typically be around 30% in all women. Recently, HPV vaccines have been developed such as Gardasil^{®} and Cervarix^{®}. However, cervical cancer remains a life-threatening disease. The cancer is unfortunately often diagnosed late since symptoms may be absent until the cancer has developed to a late stage. One possible early sign of cervical cancer is vaginal bleeding. Cervical cancer is diagnosed based on biopsy procedures. The main treatment is surgery, however, radiation and chemotherapy can be used in late stages of the disease. The prognosis of patients with cervical cancer depends on disease stage at the time of diagnosis. HPV infections may also affect other parts of the female reproductive system, such as the vagina and this infection can develop into vaginal cancer. Multiple infection sites such as vagina and cervix are possible.

Cervical intraepithelial neoplasia (CIN), also known as cervical dysplasia, is the potentially premalignant transformation and abnormal growth of squamous cells on the surface of the cervix. Corresponding thereto, vaginal intraepithelial neoplasia (VAIN), also known as vaginal dysplasia, is the potentially premalignant transformation and abnormal growth of squamous cells in the vagina, usually in the upper 1/3 of vagina (may be confluent with cervical lesions). Most cases of such dysplasia remain stable, or are eliminated by the body's immune system without intervention. However a small percentage of cases progress to become cancer, usually squamous cell carcinoma (SCC), if left untreated. The major cause of CIN, and VAIN is chronic infection of the affected organs or tissue with HPV, especially the high-risk HPV types 16 or 18.

Over 100 types of HPV have been identified. About a dozen of these HPV types appear to cause cervical dysplasia and may lead to the development of cervical cancer. The earliest microscopic change corresponding to CIN is dysplasia of the epithelial or surface lining of the cervix, which is essentially undetectable by the woman. Cellular changes associated with HPV infection, such as koilocytes, are also commonly seen in CIN. CIN is usually discovered by a screening test, the Papanicolaou or "Pap" smear, by which also VAIN can be diagnosed. The purpose of these tests is to detect the changes early, while it has not yet progressed to invasive carcinoma, and is easier to cure. An abnormal Pap smear may lead to a recommendation for colposcopy of the cervix and/or vagina during which these organs and tissues are examined under magnification. Acetic acid solution or iodine solution may be applied to the surface to improve visualization of abnormal areas. A biopsy is taken of any abnormal appearing areas since cervical and vaginal dysplasia can be diagnosed by histological examination of biopsy specimens.

Methods used to treat the above-mentioned intraepithelial neoplasia require removal or destruction of the diseased epithelial of the cervix, including the transformational zone and vagina. These methods include excision, cryocautery, electrocautery, laser cautery, LEEP (cervix), and cervical conisation. All said methods may have side effects, like (cervical) stenosis, compromised conception, cervical insufficiency with premature delivery and low-birth weight babies, infections and hemorrhage. The procedure causes patient anxiety and hence there remains a medical need for a tissue preserving treatment of such intraepithelial neoplasia. PDT has proven to be such an alternative with the treated patients showing good response rates.

In PDT of CIN, both 5-ALA and esters of 5-ALA have been used. K. Bodner et al., Anticancer Res 2003, 23(2C): 1785-1788 used a solution of 5-ALA (12% w/v) in 0.9% aqueous NaCl solution containing 1% EDTA (w/v). The 5-ALA solution was prepared just before conducting the PDT. A. Barnett et al, Int. J. Cancer, 103, 829-832 (2003) have used a 3% or 5% (w/w) solution of 5-ALA in Intrasite Gel^{®} which was prepared immediately prior to use. Intrasite Gel is a hydrogel comprising 2.3% of a modified carboxymethylcellulose (CMC) polymer together with propylene glycol (20%) as a humectant and preservative. P. Hillemanns et al., Int. J. Cancer: 81, 34-38 (1999) used 5-ALA hydrochloride which was freshly dissolved in sterile 0.9% aqueous NaCl solution at a final concentration of 20% (w/w) containing propylene glycol and adjusted at pH 5.5 using NaHCO₃. P. Soergel et al., Lasers in Surgery and Medicine 40:611-615, 2008 used 5-ALA hexyl ester which was applied in a thermogel formulation. As a thermogel base, Lutrol F-127, a bioadhesive poloxamer was used which was provided as a powder and the thermogel had to be prepared on site by adding sterile water. The 5-ALA hexyl ester containing thermogel had to be prepared freshly before application.

As seen above, freshly prepared formulations of 5-ALA and 5-ALA esters have been used for PDT of CIN due to the limited stability of these compounds, which in turn limits the shelf life of pharmaceutical products in which they are present.

A number of different strategies have been adopted to try to overcome this stability problem: formulations have been developed which exhibit enhanced stability, alternatively, pharmaceutical preparations are transported and stored in cold conditions.

WO 2010/142457 discloses semi-solid compositions for use in the treatment intraepithelial neoplasia in the female reproductive system. The semi-solid formulations (e.g. ointments, pastes, creams or gels) exhibit a remarkably enhanced stability. WO 2009/074811 discloses solid pharmaceutical products for use in PDT of cancer and non-cancerous conditions like HPV infections in the female reproductive system. Said solid pharmaceutical products may be for administration in the form of a suppository or pessary and exhibit enhanced stability. Metvix^{®}, a cream formulation containing 5-ALA methyl ester, is stored in cold conditions.

These approaches, however, have disadvantages. For example, it is not always convenient to transport and store medicines in cold conditions.

Irradiation devices have now been developed to carry out PDT for the treatment of cancer, pre-cancerous conditions and non-cancerous conditions in the cervix, i.e. HPV infections and CIN, see WO 2010/078929, Photocure ASA. The irradiation device is fully inserted into the vagina and its treatment surface covers the portio and opening of the cervix. The device is independently operable whilst inside the patient. Such device contains an area for carrying a drug, e.g. a composition comprising a photosensitizer or a precursor thereof. The device may be used in combination with the semi-solid compositions described in WO 2010/142457: the semi-solid composition is applied to the area for carrying the drug on such a device, the device plus drug is inserted into the vagina and placed at the site of treatment, e.g. over the portio.

The provision of an integrated, ready-to-use PDT device, i.e. a PDT device containing the drug as described above, would ensure that the drug is used in an accurate concentration which ensures treatment success. This is particularly important in the treatment of the majority of diseases including cancer where it can be critical that the correct and efficient dosage of therapeutic is administered. Moreover an integrated, ready-to-use PDT device would be most convenient for medical practitioners since they don't need to spend time applying the drug to the area for carrying a drug on said device before they can apply the device to the patient.

Hence there is a need for alternative formulations of 5-ALA and 5-ALA esters and thus pharmaceutical products comprising 5-ALA and 5-ALA ester for use in PDT of cancer, pre-cancerous conditions and non-cancerous conditions, preferably for use in PDT of HPV infections, e.g. HPV infections of the cervix and vagina.

We have now surprisingly found that dry compositions comprising as an active ingredient 5-ALA or a derivative thereof (e.g. an ALA ester) are suitable for use in the photodynamic treatment of cancer, pre-cancerous conditions and non-cancerous conditions. Such dry compositions may be manufactured in such a way that they form a film or a thin coat which covers/lines an area for carrying the drug contained in a light emitting device, i.e. irradiation device, which is used in the photodynamic treatment. The devices can for instance be sealed air- and moisture tight, such that the device plus drug contained in the dry composition within said device have a long shelf life at room temperature, e.g. up to 5 years. Such device plus dry composition are easy to handle by health personnel. In use, the device plus drug is placed at the site of treatment. Such site of treatment is a moist environment in a body cavity of
the human or non-human animal body, namely the
mucosa lined surface on the cervix. Upon contact of the device/area on said device which carries the dry composition comprising the active ingredient, the water and fluids contained in the mucosa react with the dry composition and result in the disintegration/dissolution of said dry composition, upon which the active ingredient is released, taken up by the cells, converted to an active photosensitizer and protoporphyrin (PpIX) is built-up. The device is activated and light is emitted for the photodynamic treatment when a therapeutically effective PpIX level is reached. Irradiation devices containing such dry compositions and such dry compositions may be used in the photodynamic diagnosis (PDD) of cancer, pre-cancerous conditions and non-cancerous conditions.

The invention provides an irradiation device according to claim 1.

Whilst it is preferred that the pharmaceutical compositions herein described should be substantially free from any solvent (e.g. water), these may nonetheless contain residual solvent. The term "dry" should thus be construed accordingly. Preferred compositions are those which are substantially solvent free, for example those prepared by any of the processes herein described. Such processes need not involve the use of any subsequent means to further reduce or eliminate any residual solvent. As noted above, the use of dry compositions ensures that the device (which includes the composition) has a long shelf life. Packaging and storage of the device in a moisture-free environment is also important.
The term "pre-cancerous condition" denotes a disease, syndrome, or finding that, if left untreated, may lead to cancer, e.g. dysplasia and neoplasia.
The term "non-cancerous conditions" includes abnormal lesions with no or low malignant potential such as hyperplasia and low-grade lesions, infections such as viral, bacterial or fungal infections, preferably HPV infection, or inflammation.
The term "active ingredient" denotes 5-ALA and pharmaceutically acceptable salts thereof, precursors of 5-ALA and pharmaceutically acceptable salts thereof and derivatives of 5-ALA and phannaceutically acceptable salts thereof.
The term "5-ALA" denotes 5-aminolevulinic acid, i.e. 5-amino-4-oxo-pentanoic acid.

The term "precursor of 5-ALA" denotes compounds which are converted metabolically to 5-ALA and thus are essentially equivalent thereto. Thus the term "precursor of 5-ALA" covers biological precursors for protoporphyrin in the metabolic pathway for haem biosynthesis.
The term "derivative of 5-ALA" denotes chemically modified 5-ALA, i.e. 5-ALA having undergone a chemical derivation such as substitution of a chemical group or addition of a further chemical group to modify or change any of its physicochemical properties such as solubility or lipophilicity. Chemical derivation is preferably carried out at the carboxy group of 5-ALA, at the amino group of 5-ALA or at the keto group of 5-ALA, more preferably at the carboxy group or the amino group of 5-ALA. Preferred derivatives include esters, amides and ethers of 5-ALA, most preferred 5-ALA esters.
The term "pharmaceutically acceptable salt" denotes a salt that is suitable for use in the dry pharmaceutical product and which fulfils the requirements related to for instance safety, bioavailability and tolerability (see for instance P. H. Stahl et al. (eds.) Handbook of Pharmaceutical Salts, Publisher Helvetica Chimica Acta, Zurich, 2002)

The irradiation device of the invention is a device for full and secure insertion into the vagina, and is independently operational while located in the vagina. The irradiation device comprises, in addition to the area for carrying the pharmaceutical composition, a housing adapted to be fully inserted and secured in the vagina, the housing enclosing a LED lamp system and a power source for powering the LED lamp system. Such devices are for instance described in WO2010/078929.

The device further comprises a treatment surface, i.e. a surface where the LED lamp system is arranged to emit radiation from and which directs and/or focuses irradiation onto the cervix inside of the vagina that is in need of
photodynamic treatment. The area for carrying the dry pharmaceutical composition is the treatment area itself.

As an example, the device is preferably shaped like the devices in Figs. 1-3 and 6-7 of WO 2010/078929, i.e. comprising an upper housing portion which is approximately frustoconical in shape and whose front end forms a treatment surface which is shaped so as to cover, in use, the portio and the opening of the cervix, thus providing irradiation to said areas. The treatment surface forms a reservoir for housing the dry pharmaceutical composition.
In one embodiment, the dry pharmaceutical composition is in the form of a powder, i.e. a dry, bulk solid composed of a large number of very fine particles, more preferably in the form of a compressed powder, i.e. having lost its ability to flow. In another embodiment, the dry pharmaceutical composition is in the form of a cake, i.e. dry bulk solid formed into a small block. In a preferred embodiment, the dry pharmaceutical composition is in the form of a film, i.e. one or more (thin) layers of dry/dried material, preferably a relatively homogeneous film which covers substantially the whole treatment surface. In a further preferred embodiment, this film is well attached and stays well attached to the treatment surface, i.e. is relatively stable under mechanical stress which may occur during transport and shipment of the irradiation device comprising the dry pharmaceutical composition.

The dry pharmaceutical composition may be obtained and deposited onto the device by any method resulting in a deposition of the dry pharmaceutical composition at the area for carrying it (hereinafter also denoted "deposition area").

In one embodiment, the dry pharmaceutical composition may be obtained as a film by film coating processes known in the art, preferably by dip-coating or spray-coating.

Dip-coating techniques can be described as a process where the substrate to be coated is immersed in a liquid and then withdrawn with a well-defined withdrawal speed under controlled temperature and atmospheric conditions. The coating thickness is mainly defined by the withdrawal speed, by the solid content and the viscosity of the liquid. In a dip-coating process, the deposition area of the device according to the invention is immersed in a liquid, i.e. a solution or dispersion of the active ingredient and optionally one or more polymers and/or other pharmaceutically acceptable excipients in one or more suitable solvents. The deposition area is withdrawn from the liquid whereby the liquid is deposited. The withdrawing is preferably carried out at a constant speed to achieve a uniform coating. Concomitantly, excess liquid is drained from the surface of the deposition area. The solvent evaporates from the liquid, forming a film. This process can be accelerated by providing heat. For volatile solvents such as lower alcohols, evaporation starts already during the deposition and drainage steps. Dip-coating is especially suitable for devices comprising an elongated housing including a treatment surface about its outer circumferential surface, e.g. devices shown in Figs. 5A and 5B of WO 2010/078929. The device containing the so-obtained dry pharmaceutical composition is sealed in an airtight/moisture tight bag. Alternatively, the area for carrying the dry pharmaceutical composition is covered/sealed such that this is moisture tight.

Spray-coating involves atomizing or aerosolizing of the liquid, i.e. a solution or dispersion of the active ingredient and optionally one or more polymers and/or optionally other pharmaceutically acceptable excipients in one or more suitable solvents, by a spray gun and coating the deposition area of the device according to the invention. Preferably, the spray gun can be adjusted horizontally, vertically and angularly and it can be swiveled too. Heaters can be used to accelerate evaporation of the solvent(s). Spray-coating is suitable for both devices including a treatment surface about their outer circumferential surface or a reservoir. The device or, if the device is comprised of modules, the module which contains the so-obtained dry pharmaceutical composition is sealed in an airtight/moisture tight bag. Alternatively, the area for carrying the dry pharmaceutical composition is covered/sealed such that this is moisture tight.

In another embodiment, the dry pharmaceutical composition is obtained by solvent evaporation. A liquid is prepared by dissolving or suspending the active ingredient and optionally one or more polymers and/or optionally other pharmaceutically acceptable excipients in one or more suitable solvents, preferably solvents with a low boiling point like for instance lower alcohols, ethers etc. The so-obtained liquid is applied to the deposition area, e.g. filled in the reservoir. The device or, if the device is comprised of modules, the module which contains the deposition area, is exposed to heat for a period which is long enough to achieve complete evaporation of the solvent(s). During that period, the device may be moved, e.g. rotated to promote evaporation and to ensure a homogeneous distribution of the liquid. Depending on the parameters of the evaporation like temperature and humidity, and the amount of liquid applied to a given deposition area, the dry pharmaceutical composition is obtained as a film or cake. The device or, if the device is comprised of modules, the module which contains the so-obtained dry pharmaceutical composition is sealed in an airtight/moisture tight bag. Alternatively, the area for carrying the dry pharmaceutical composition is covered/sealed such that this is moisture tight.

In a preferred embodiment, the dry pharmaceutical composition is obtained by lyophilization. Briefly, in general the lyophilization process consists of three stages: freezing, primary drying and secondary drying. A liquid, i.e. solution or suspension of the compound(s) to be lyophilized is first frozen. Usually freezing temperatures are between -50 to -80°C, depending on which solvent(s) is used. During the primary drying phase, the pressure is lowered and enough heat is supplied to the frozen liquid for the solvent, usually water, to sublimate. In the secondary drying phase, unfrozen solvent molecules are removed. For obtaining the dry pharmaceutical composition according to the invention by lyophilization, a liquid is prepared by dissolving or suspending the active ingredient and optionally one or more polymers and/or and optionally other pharmaceutically acceptable excipients in a suitable solvent, usually water. However, it is also possible to use mixtures of solvents, e.g. water and alcohols such as ethanol. The so-obtained liquid is applied to the deposition area, e.g. filled in the reservoir. The device may be cooled and/or moved, e.g. rotated, during the application of the liquid. The device or, if the device is comprised of modules, the module which contains the deposition area, is frozen, for instance quickly frozen to avoid the formation of larger crystals. The device or module is then lyophilized as described before. The dry pharmaceutical composition is typically obtained in the form of a cake, a powder (which can be further compressed) or preferably a film. The device or, if the device is comprised of modules, the module which contains the so-obtained dry pharmaceutical composition is sealed in an airtight/moisture tight bag. Alternatively, the area for carrying the dry pharmaceutical composition is covered/sealed such that this is moisture tight.
Specific equipment for dip-coating, spray-coating and lyophilization is commercially available.

The use of 5-ALA and derivatives thereof, e.g. 5-ALA esters in PDT and PDD is well known in the scientific and patent literature, see, for example, WO 2006/051269, WO 2005/092838, WO 03/011265, WO 02/09690, WO 02/10120, WO 2003/041673 and US 6,034,267.

All such derivatives of 5-ALA and their pharmaceutically acceptable salts are suitable for use in the methods herein described.

The synthesis of 5-ALA is known in the art. Further, 5-ALA and pharmaceutically acceptable salts thereof are commercially available, for instance from Sigma Aldrich.

The 5-ALA derivatives useful in accordance with the invention may be any derivative of 5-ALA capable of forming protoporphyrins, e.g. PpIX or a PpIX derivative *in vivo.* Typically, such derivatives will be a precursor of PpIX or of a PpIX derivative, e.g. a PpIX ester in the biosynthetic pathway for haem and which are therefore capable of inducing an accumulation of PpIX following administration *in vivo.* Suitable precursors of PpIX or PpIX derivatives include 5-ALA prodrugs which might be able to form *5-ALA in vivo* as an intermediate in the biosynthesis of PpIX or which may be converted, e.g. enzymatically, to porphyrins without forming 5-ALA as an intermediate. 5-ALA esters and pharmaceutically acceptable salts thereof, are among the preferred compounds for use in the invention described herein.

Esters of 5-ALA which are optionally N-substituted are preferred for use in the invention. Those compounds in which the 5-amino group is unsubstituted, i.e. 5-ALA esters, are particularly preferred. Such compounds are generally known and described in the literature see, for example, WO 96/28412 and WO 02/10120 to Photocure ASA, WO 03/041673 and in N. Fotinos et al., Photochemistry and Photobiology 2006: 82, 994-1015.

Esters resulting from a reaction of 5-ALA with unsubstituted or substituted alkanols, i.e. alkyl esters and substituted alkyl esters, and pharmaceutically acceptable salts thereof, are especially preferred derivatives of 5-ALA for use in the invention. Examples of such preferred 5-ALA esters include those of general formula I and pharmaceutically acceptable salts thereof:

R²₂N-CH₂COCH₂-CH₂CO-OR¹ (I)

wherein
R¹ represents a substituted or unsubstituted alkyl group; and
R² each independently represents a hydrogen atom or a group R¹

As used herein, the term "alkyl", unless stated otherwise, includes any long or short chain, cyclic, straight-chained or branched saturated or unsaturated aliphatic hydrocarbon group. Unsaturated alkyl groups may be mono- or polyunsaturated and include both alkenyl and alkynyl groups. Unless stated otherwise, such alkyl groups may contain up to 40 carbon atoms. However, alkyl groups containing up to 30 carbon atoms, preferably up to 10, particularly preferably up to 8, especially preferably up to 6 carbon atoms are preferred.

In compounds of formula I, the R¹ groups are substituted or unsubstituted alkyl groups. If R¹ is a substituted alkyl group, one or more substituents are either attached to the alkyl group and/or interrupt the alkyl group. Suitable substituents that are attached to the alkyl group are those selected from hydroxy, alkoxy, acyloxy, alkoxycarbonyloxy, amino, aryl, nitro, oxo, fluoro, -SR³, -NR³₂ and -PR³₂, wherein R³ is a hydrogen atom or a C₁₋₆ alkyl group. Suitable substituents that interrupt the alkyl group are those selected from -O-, -NR³-, -S- or -PR³.

In a preferred embodiment, R¹ is an alkyl group substituted with one or more aryl substituents, i.e. aryl groups. Preferably, R¹ is an alkyl group substituted with one aryl group.

As used herein, the term "aryl group" denotes an aromatic group which may or may not contain heteroatoms like nitrogen, oxygen or sulfur. Aryl groups which do not contain heteroatoms are preferred. Preferred aryl groups comprise up to 20 carbon atoms, more preferably up to 12 carbon atoms, for example, 10 or 6 carbon atoms. Preferred embodiments of aryl groups are phenyl and naphthyl, especially phenyl. Further, the aryl group may optionally be substituted by one or more, more preferably one or two, substituents. Preferably, the aryl group is substituted at the meta or para position, most preferably the para position. Suitable substituents include halo alkyl, e.g. trifluoromethyl, alkoxy, preferably alkoxy groups containing 1 to 6 carbon atoms, halo, e.g. iodo, bromo, chloro or fluoro, preferably chloro and fluoro, nitro and C₁₋₆ alkyl, preferably C₁₋₄ alkyl. Preferred C₁₋₆ alkyl groups include methyl, isopropyl and t-butyl, particularly methyl. Particularly preferred aryl substituents are chloro and nitro. However, still more preferably the aryl group is unsubstituted.

Preferred such aryl substituted R¹ groups are benzyl, 4-isopropylbenzyl, 4-methylbenzyl, 2-methylbenzyl, 3-methylbenzyl, 4-[t-butyl]benzyl, 4-[trifluoromethyl]benzyl, 4-methoxybenzyl, 3,4-[di-chloro]benzyl, 4-chlorobenzyl, 4-fluorobenzyl, 2-fluorobenzyl, 3-fluorobenzyl, 2,3,4,5,6-pentafluorobenzyl, 3-nitrobenzyl, 4-nitrobenzyl, 2-phenylethyl, 4-phenylbutyl, 3-pyridinyl-methyl, 4-diphenyl-methyl and benzyl-5-[(1-acetyloxyethoxy)-carbonyl]. More preferred such R¹ groups are benzyl, 4-isopropylbenzyl, 4-methylbenzyl 4-nitrobenzyl and 4-chlorobenzyl. Most preferred is benzyl.

If R¹ is a substituted alkyl group, one or more oxo substituents are preferred. Preferably, such groups are straight-chained C₄₋₁₂ alkyl groups which are substituted by one or more oxo groups, preferably by one to five oxo groups. The oxo groups are preferably present in the substituted alkyl group in an alternating order, i.e. resulting in short polyethylene glycol substituents. Preferred examples of such groups include 3,6-dioxa-l-octyl and 3,6,9-trioxa-l-decyl. In another preferred embodiment, R¹ is an alkyl group interrupted by one or more oxygen atoms (ether or polyether group), preferably a straight-chained C₄₋₁₂ alkyl and more preferably a straight-chained C₆₋₁₀ alkyl group being interrupted by 1 to 4 oxygen atoms, more preferably a straight-chained polyethylene glycol group (-(CH₂)₂-O-)ₙ with n being an integer of from 1 to 5.

If R¹ is an unsubstituted alkyl group, R¹ groups that are saturated straight-chained or branched alkyl groups are preferred. If R¹ is a saturated straight-chained alkyl group, C₁₋₁₀ straight-chained alkyl group are preferred. Representative examples of suitable straight-chained alkyl groups include methyl, ethyl, n-propyl, n-butyl, n-pentyl, n-hexyl and n-octyl. Particularly preferred are C₁₋₆ straight-chained alkyl group, most particularly preferred are methyl and n-hexyl. If R¹ is a saturated branched alkyl group, such branched alkyl groups preferably consist of a stem of 4 to 8, preferably 5 to 8 straight-chained carbon atoms and said stem is branched by one or more C₁₋₆ alkyl groups, preferably C₁₋₂ alkyl groups. Examples of such saturated branched alkyl groups include 2-methylpentyl, 4-methylpentyl, 1-ethylbutyl and 3, 3-dimethyl-1-butyl.

In compounds of formula I, each R² independently represents a hydrogen atom or a group R¹. Particularly preferred for use in the invention are those compounds of formula I in which at least one R² represents a hydrogen atom. In especially preferred compounds each R² represents a hydrogen atom.

Preferably, compounds of formula I and pharmaceutically acceptable salts thereof are used as active ingredients in the dry pharmaceutical compositions of the invention, wherein R¹ is methyl or hexyl, more preferably n-hexyl and both R² represent hydrogen, i.e. 5-ALA methyl ester, 5-ALA hexyl ester and pharmaceutically acceptable salts thereof, preferably the HCl salts. The preferred compound for use as active ingredients in the dry pharmaceutical compositions of the invention is 5-ALA hexyl ester and pharmaceutically acceptable salts thereof, preferably the HCl salt or sulfonic acid salts or sulfonic acid derivative salts.

5-ALA esters and pharmaceutically acceptable salts thereof for use in the invention may be prepared by any conventional procedure available in the art, e.g. as described in WO 96/28412, WO 02/10120, WO 03/041673 and in N. Fotinos et al., Photochemistry and Photobiology 2006: 82, 994-1015 and the cited literature references therein. Briefly, 5-ALA esters may be prepared by reaction of 5-ALA with the appropriate alcohol in the presence of a catalyst, e.g. an acid. Pharmaceutically acceptable salts of 5-ALA esters may be prepared as described hereinbefore by reaction of a pharmaceutically acceptable 5-ALA salt, e.g. 5-ALA hydrochloride with the appropriate alcohol. Alternatively compounds for use in the invention like 5-ALA methyl ester or 5-ALA hexyl ester may be available commercially, e.g. from Photocure ASA, Norway.

The 5-ALA esters for use in the invention may be in the form of a free amine, e.g. -NH₂, -NHR² or -NR²R² or preferably in the form of a pharmaceutically acceptable salt. Such salts preferably are acid addition salts with pharmaceutically acceptable organic or inorganic acids. Suitable acids include, for example, hydrochloric, nitric, hydrobromic, phosphoric, sulfuric, sulfonic and sulfonic acid derivatives, the salts of ALA-esters and the latter acids are described in WO2005/092838 to Photocure ASA. A preferred acid is hydrochloride acid, HCl. Further preferred acids are sulfonic acid and sulfonic acid derivatives. Procedures for salt formation are conventional in the art and are for instance described in WO2005/092838.

In a preferred embodiment, the dry pharmaceutical composition comprises:
a) a 5-ALA ester of formula I or a pharmaceutically acceptable salt thereof:

   R²₂N-CH₂COCH₂-CH₂CO-OR¹ (I)

   wherein
   R¹ represents a substituted or unsubstituted alkyl group; and
   R² each independently represents a hydrogen atom or a group R¹;
b) one or more polymers which have good film-forming properties and/or good gel-forming properties; and
c) optionally other pharmaceutically acceptable excipients.

In a further preferred embodiment, R¹ of formula (I) represents an unsubstituted alkyl group, preferably an unsubstituted saturated straight-chained or branched alkyl group, more preferably an unsubstituted saturated straight-chained C₁₋₁₀ alkyl group. Most preferred, said 5-ALA ester is 5-ALA hexyl ester and in a further preferred embodiment, said pharmaceutically acceptable salt of 5-ALA hexyl ester is the HCl salt or a sulfonic acid salt or sulfonic acid derivative salt, such as mesylate, tosylate or napsylate.

Dry pharmaceutical compositions consisting essentially (e.g. consisting only) of the active ingredient are preferably obtained by lyophilizing a liquid (solution or suspension) of the active agent in a suitable solvent, preferably water. Usually, a cake or powder is obtained, the latter may be compressed.

The active ingredient is released from the dry pharmaceutical composition in a moist environment, i.e. upon contact with a mucosa lined surface such as the lining of the the cervix.

According to the invention, one or more polymers and optionally other pharmaceutically acceptable excipients are present in the dry pharmaceutical composition. Preferred one or more polymers are polymers which have good film-forming properties and/or good gel forming properties. Preferred other pharmaceutically acceptable excipients are selected from one or more of the following compounds: plasticizers, coloring agents and thickening agents. Other pharmaceutically acceptable excipients which may be present in the dry pharmaceutical composition are disintegrants, mucoadhesive agents, surface penetration enhancing agents and chelating agents.

If one or more polymers and/or pharmaceutically acceptable excipients are present in the dry pharmaceutical composition, the active ingredient may be present in the range of 0.25 to 50%, for example 0.5 to 30%, such as 0.5 to 15% or 1 to 10% or 1 to 7% by weight of the total weight of the dry pharmaceutical composition. Alternatively, if only one or more polymers are additionally present in the dry pharmaceutical composition, the active ingredient may be present in the range of 50 to 99%, for example 60 to 91% or 75 to 90% by weight of the total weight of the dry pharmaceutical composition. By having a high amount of active ingredient compared to the amount of the one or more polymer, the liquid which is used to deposit the composition is less viscous and thus easier to handle and process. In another embodiment if one or more polymers and pharmaceutically acceptable excipients selected from plasticizers are present in the dry pharmaceutical composition, the active ingredient may be present in the range of 15 to 85%, for example 20 to 80% such as 25 to 78% or 26 to 60% by weight of the total weight of the dry pharmaceutical composition.
All one or more polymers and pharmaceutically acceptable excipients should be non-toxic, non-irritant and devoid of leachable impurities. They should be inert towards the active ingredient, i.e. should not promote its degradation. One or more of each phannaceutically acceptable excipient compound may be used, e.g. one or more plasticizers, one or more coloring agents etc.

The one or more polymers for use in the dry pharmaceutical composition can be natural, semi-natural, i.e. derivatives of natural polymers which are obtained by a chemical reaction, or synthetic polymers; they may be homopolymers or copolymers.

Preferably, polymers are used which have good film-forming properties, i.e. which form - together with the active ingredient - a film when deposited to the area intended for carrying the pharmaceutical composition on the device. A preferred group of such polymers are starch, cellulose and derivatives of starch and cellulose. Preferred starch derivatives are starch acetate and carboxymethyl starches, preferably with an amylose content of at least 18% by weight. One preferred cellulose is microcrystalline cellulose. Other preferred cellulose derivatives are cellulose ethers such as methylcellulose, ethylcellulose, hydroxyethylcellulose, hydroxypropylcellulose, hydroxypropylmethylcellulose, hydroxypropylethylcellulose and carboxymethylcellulose. Such polymers may be used in combination with other polymers, e.g. ethylcellulose with hydroxypropylmethylcellulose. Other preferred cellulose derivatives are cellulose acetate phthalate and nitrocellulose. Further preferred polymers are rosin and rosin esters. Another preferred group of polymers are (meth) acrylate polymers and copolymers. The use of "meth" as a prefix in parenthesis indicates, in accordance with common practice, that the polymer molecule is derived from monomers having the carbon atom skeleton of either or both of acrylic acid and methacrylic acid. Such polymers and copolymers are e.g. based on methylmethacrylate, ethylacrylate, methacrylic acid and trimethylammonioethylmethacrylate chloride, e.g. anionic and cationic polymers of methacrylic acid, copolymers of methacrylates, copolymers of acrylates and methacrylates, copolymers of ethylacrylates and methylmethacrylates. Other preferred polymers are polyvinyl acetate phthalate. In a more preferred embodiment, cellulose and cellulose derivatives, especially cellulose ethers, are used as one or more polymers in the dry pharmaceutical compositions according to the invention.

In another embodiment, polymers with good gel-forming properties are used, i.e. which form - together with the active ingredient - gels on contact with water and fluids of mucosa lined surfaces. Preferred such polymers are gums, preferably gellan gum, xanthan gum and camageenan. Other preferred polymers are chitin, chitosan and chitosan derivatives such as chitosan salts (hydrochloride, lactate, aspartate, glutamate) and N-acetylated chitosan or N-alkylated chitosan. Yet other preferred polymers are pectin, alginates, e.g. sodium alginate, pullulan, hyaluronic acid and derivatives thereof.

Preferably, in yet another embodiment, polymers with good film-forming properties and good gel-forming properties are used, e.g. cellulose ethers like methylcellulose, ethylcellulose, gellan gum, chitosan and chitosan derivatives, pullulan, alginates, hyaluronic acid, derivatives of hyaluronic acid or carrageenan. Preferred such polymers are cellulose ethers like methylcellulose, ethylcellulose, hydroxyethylcellulose, hydroxypropylcellulose, hydroxypropylmethylcellulose, hydroxypropylethylcellulose and carboxymethylcellulose and chitosan and chitosan derivatives.

Polymers with good film-forming properties are preferably used if the dry pharmaceutical composition should form a film.

The polymers may be water soluble or insoluble in water. In a preferred embodiment, water soluble polymers are used.

Thus in a preferred embodiment said one or more polymers are selected from cellulose ethers, gellan gum, chitosan, chitosan derivatives, pullulan,

alginates, hyaluronic acid, derivatives of hyaluronic acid and carrageenan.

In a further preferred embodiment said one or more polymers are selected from chitosan, chitosan derivatives and cellulose

The one or more polymers may conveniently be provided in a concentration range of 50 to 99.75%, for example 70 to 99.5%, e.g. 85 to 99.5%, or 90 to 99% or 93 to 99% by weight of the total weight of the dry pharmaceutical composition. Alternatively, if only one or more polymers are additionally present in the dry pharmaceutical composition, the one or more polymers may be present in the range of 1 to 50 %, for example 9 to 40% or 10 to 25% by weight of the total weight of the dry pharmaceutical composition. By having a high amount of active ingredient compared to the amount of the one or more polymer, the liquid which is used to deposit the composition is less viscous and thus easier to handle and process. In another embodiment if one or more polymers and pharmaceutically acceptable excipients selected from plasticizers are present in the dry pharmaceutical composition, the one or more polymers may be present in the range of 20 to 65%, for example 25 to 62% such as 30 to 55% or 40 to 54% by weight of the total weight of the dry pharmaceutical composition.

In an embodiment the optional pharmaceutically acceptable excipients are present and are selected from plasticizers, coloring agents, thickening agents, disintegrants, mucoadhesive agents, surface penetration enhancing agents and/or chelating agents.

Other pharmaceutically acceptable excipients which may be present in the dry pharmaceutical composition are plasticizers. In general their use is to reduce the glass transition temperature of a polymer making it more elastic and deformable, i.e. flexible. Hence they may be present in the dry pharmaceutical composition if one or more polymers are present, preferably if one or more film-forming polymers are present. Plasticizers are preferably chosen in such a way that they work well with the given polymer(s). In one embodiment, suitable plasticizers are acting as a good solvent for the polymer(s) in question. In another embodiment, if water soluble polymers are used, the plasticizer is preferably a water miscible compound. Suitable plasticizers are low molecular weight polyethylene glycols, phthalate derivatives like dimethyl, diethyl and dibutyl phthalate, citrate esters such as triethyl, tributyl and acetyl citrate, dibutyl sebacate, camphor, triacetin, oils and glycerides such as castor oil, acetylated monoglycerides and fractionated coconut oil. Glycerol and propylene glycol are also common plasticizers, they should however not be used if the dry pharmaceutical composition contains as an active ingredient lower alkyl ALA esters or salts thereof, such as C₁-C₈-alkyl ALA esters since they may promote degradation of such active ingredients.

If present in the dry pharmaceutical composition, the plasticizers may conveniently be provided in a concentration range of 1 to 30%, for example 5 to 20% or 7 to 15% by weight of the total polymer weight. Alternatively, the plasticizers may be
provided in a higher concentration range, for instance in a concentration range of 10 to 175%, or 35 to 150% or 37 to 80% by weight of the total polymer weight.

Other pharmaceutically acceptable excipients which may be present in the dry pharmaceutical composition are coloring agents, such as synthetic dyes or pigments, e.g. titanium dioxide or yellow iron oxide. Pigments usually decrease the permeability of the dry pharmaceutical composition to water vapor and oxygen and may thus increase its shelf life. Further, they contribute to the total solids of the liquid used to obtain the dry pharmaceutical composition without significantly contributing to its viscosity. Thus faster processing time by virtue of more rapid drying is possible, which is particularly significant for aqueous based liquids used in spray-coating.

If present in the dry pharmaceutical composition, the coloring agents may conveniently be provided in a concentration range of 0.1 to 20%, for example 0.5 to 10% or 1 to 5% by weight of the total dry pharmaceutical composition.

Other pharmaceutically acceptable excipients which may be present in the dry pharmaceutical composition are thickening agents. Such agents swell as they absorb liquid and thus may be used to improve the viscosity and consistency of the liquid which is used to obtain the dry pharmaceutical composition. Preferably, thickening agents are used in liquids which are employed in dip-coating. The choice of thickening agents is dependent on whether the liquid is an aqueous or aqueous based liquid or whether non-aqueous solvents are used to form the liquid. Some of the aforementioned polymers have thickening properties, e.g. gums like guar gum, cellulose derivatives like carboxymethylcellulose and (meth)acrylates. Other suitable thickening agents are polyacrylic acids (carbomer) or wax or a waxy solids e.g. solid fatty alcohols or solid fatty acids.

If present in the dry pharmaceutical composition, the thickening agents may conveniently be provided in such an amount that the desired viscosity of the liquid described above is obtained. The actual amount will depend on the one or more solvent said liquid comprises and the nature of the thickening agent.

Other pharmaceutically acceptable excipients which may be present in the dry pharmaceutical composition are disintegrants. Generally, disintegrants aid in the break up of the dry pharmaceutical composition when it is put into a moist environment. Some of the aforementioned polymers do exhibit disintegrant properties, e.g. certain celluloses, starch and derivatives thereof. If these polymers are present, there may not be a need or desire to add any further disintegrants. More effective disintegrants are referred to as superdisintegrants. Those include for instance alginic acid, croscarmellose, crospovidone and sodium starch glycolate. Such compounds swell when they come in contact with fluids but they do not form a gel which would decrease their disintegration properties.

If present in the dry pharmaceutical composition, the disintegrants may conveniently be provided in a concentration range of 0.1 to 10% by weight of the total weight of the dry pharmaceutical composition, for example 0.25 to 5% or 0.5 to 4% by weight.

The dry pharmaceutical compositions may further comprise one or more mucoadhesive agents. The term "mucoadhesive agent" denotes a compound which exhibits an affinity for a mucosa surface, i.e. which adheres to that surface through the formation of bonds which are generally non-covalent in nature, whether binding occurs through interaction with the mucous and/or the underlying cells.

The mucoadhesive agent is preferably a compound that is not degraded by the pH or bacterial conditions of the mucosa lined surfaces, i.e. the acidic environment in the vagina which is due to the presence of lactic acid or the bacterial and non-bacterial enzymes present in the vagina and on the cervix or the bacteria present in the rectum.

Suitable mucoadhesive agents may be natural or synthetic compounds, polyanionic, polycationic or neutral, water-soluble or water-insoluble, but are preferably large, e.g. having a molecular weight of 500 kDa to 3000 kDa, e.g. 1000 kDa to 2000 kDa, water-insoluble cross-linked, e.g. containing 0.05 % to 2 % cross-linker by weight of the total polymer, prior to any hydration, water-swellable polymers capable of forming hydrogen bonds. Preferably such mucoadhesive compounds have a mucoadhesive force greater than 100, especially preferably greater than 120, particularly greater than 150, expressed as a percent relative to a standard *in vitro,* as assessed according to the method of Smart et al., 1984, J. Pharm. Pharmacol., 36, pp 295-299.

Some of the aforementioned polymers exhibit mucoadhesive properties, for instance gums like guar gum, chitosan and chitosan derivatives, pullulan, sodium alginate or hyaluronic acid. If these polymers are present, there may not be a need or desire to add any further mucoadhesive agents.

Suitable mucoadhesive agents are selected from polysaccharides, preferably dextran, pectin, amylopectin or agar; gums, preferably guar gum or locust bean gum; salts of alginic acid, e.g. magnesium alginate; poly(acrylic acid) and crosslinked or non-crosslinked copolymers of poly(acrylic acid) and derivatives of poly(acrylic acid) such as salts and esters like for instance carbomer (carbopol).

When present, the mucoadhesive agent may conveniently be provided in a concentration range of 0.05 to 30%, e.g. about 1 to 25 % by weight of the total weight of the dry pharmaceutical composition.

The dry pharmaceutical composition may further comprise one or more surface penetration enhancing agents. Such agents may have a beneficial effect in enhancing the photosensitizing effect the active ingredient, i.e. of 5-ALA, the derivative of 5-ALA or the precursor of 5-ALA present in the dry pharmaceutical composition.

Preferred surface penetration enhancing agents are chelators (e.g. EDTA), surfactants (e.g. sodium dodecyl sulfate), non-surfactants, bile salts (sodium deoxycholate), fatty alcohols e.g. oleylalcohol, fatty acids e.g. oleic acid and esters of fatty acids and alcohol, e.g. isopropylmyristate.

When present, the surface penetration enhancing agent may conveniently be provided in a concentration range of 0.2 to 20% by weight of the total weight of the dry pharmaceutical composition, e.g. about 1 to 15% or 0.5 to 10% by weight of the total weight of the dry pharmaceutical composition.

The dry pharmaceutical composition may further comprise one or more chelating agents. Such agents may also have a beneficial effect in enhancing the photosensitizing effect of 5-ALA, the derivative of 5-ALA or the precursor of 5-ALA present in the pharmaceutical compositions of the invention.

Chelating agents may, for example, be included in order to enhance the accumulation of PpIX since the chelation of iron by the chelating agent prhomogeneousts its incorporation into PpIX to form haem by the action of the enzyme ferrochelatase, thereby leading to a build up of PpIX. The photosensitizing effect is therefore enhanced.

Suitable chelating agents are aminopolycarboxylic acids and any of the chelants described in the literature for metal detoxification or for the chelation of paramagnetic metal ions in magnetic resonance imaging contrast agents. Particular mention may be made of EDTA, CDTA (cyclohexane triamine tetraacetic acid), DTPA and DOTA and well known derivatives and analogues thereof. EDTA and DTPA are particularly preferred. Other suitable chelating agents are desferrioxamine and siderophores and they may be used alone or in conjunction with aminopolycarboxylic acid chelating agents such as EDTA.

Some of the above-mentioned chelating agents do also exhibit surface penetration assisting agent properties, e.g. EDTA.

Where present, the chelating agent may conveniently be used at a concentration of 0.01 to 12%, e.g. 0.1 to 5% by weight based on the total weight of the dry pharmaceutical composition.

The dry pharmaceutical composition may further comprise one or more pharmaceutically acceptable excipients which are different from the aforementioned excipients. Such excipients are for instance surfactants, emulsifiers, preferably nonionic or cationic emulsifiers, fillers, binders, spreading agents, stabilizing agents or preservatives. The skilled man will be able to select suitable excipients based on their purpose. Common excipients that may be used in the pharmaceutical products herein described are listed in various handbooks (e.g. D.E. Bugay and W.P. Findlay (Eds) Pharmaceutical excipients (Marcel Dekker, New York, 1999), E-M Hoepfner, A. Reng and P.C. Schmidt (Eds) Fiedler Encyclopedia of Excipients for Pharmaceuticals, Cosmetics and Related Areas (Edition Cantor, Munich, 2002) and H.P. Fielder (Ed) Lexikon der Hilfsstoffe fur Pharmazie, Kosmetik und angrenzende Gebiete (Edition Cantor Aulendorf,1989)).

All of the above-mentioned pharmaceutically acceptable excipients are well known in the art and are commercially available from various manufacturers.

In order to achieve a full release of the active ingredient, the one or more polymers or other pharmaceutically acceptable excipients need to be chosen in such a way that they are either dissolved upon contact with the mucosa-lined surfaces or at least disintegrate to allow release of the active ingredient. The excipients, especially any polymers present thus need to be dissolved or disintegrated at the given pH on said mucosa-lined surfaces. The pH inside the vagina and on the surface of the cervix is about 3.8-4.5 while the pH in the rectum is around 7.9. The pH in the nasal cavity is about 6.3-6.4.

The release profile (immediate/quick, sustained and delayed release) and the residence time of the pharmaceutical composition according to the invention at the area in need to treatment can be influenced by the choice of the polymer as well. A quick release of the active ingredient may be preferred if a comparably high concentration of the active ingredient at the site of treatment is desired. A delayed release of the active ingredient may be preferred if a low concentration of the active ingredient at the site of treatment is desired. The moistened pharmaceutical composition is preferably in a form that ensures a long residence time, e.g. in the form of a gel rather than in a dissolved liquid form.

How the release profile and residence time can be influenced is illustrated with the polymer chitosan: chitosan is a weak base with a pKa value of about 6.2-7.0 and, therefore, it is insoluble at neutral and alkaline pH values. Hence a dry pharmaceutical composition containing chitosan as a polymer may be useful to achieve quick release of the active ingredient in an acidic environment, e.g. in the vagina and on the cervix. On the other hand a dry pharmaceutical composition containing chitosan as a polymer may be useful to achieve sustained/delayed release of the active ingredient in an environment with a pH above 7, e.g. in the rectum: chitosan shows a swelling ability in contact with the mucosa of the rectum and the active ingredient is slowly released. The solubility of chitosan can be influenced by derivatization: its solubility at near neutral pH can be improved by introducing hydrophilic functional groups such as carboxymethyl or by selective N-acetylation.

Chitosan is a mucoadhesive agent but its residence time on the vaginal mucosa was shown to be enhanced by introduction of thiol groups which lead to a more controlled release of the active ingredient (see C. E. Kast, J Control Release 2002, vol. 81, 354-374.). The release profile/residence time is further influenced by the molecular weight of the used chitosan/chitosan derivative, the amount of chitosan in the liquid which is used to prepare the dry pharmaceutical composition and by the ratio between the active ingredient and chitosan. A dry pharmaceutical composition obtained from liquid with a low amount of chitosan usually shows faster release of the active ingredient because of its low consistency/low compactness. In dry pharmaceutical compositions with a hydrophilic active ingredient, e.g. a short alkyl 5-ALA ester, both the chitosan molecules and the active ingredient molecules compete for the water molecules.

The dry pharmaceutical compositions comprising an active ingredient selected from 5-ALA, a precursor of 5-ALA or a derivative of 5-ALA, and one or more polymers selected from chitosan or chitosan derivatives are preferably obtained as follows:
Non-water soluble chitosan is dissolved in hydrochloric acid (0.1 M) which is evaporated to dryness. The residual or alternatively a water soluble derivative of chitosan is dissolved in water. The active ingredient is added and dissolved in the chitosan solution to result in a liquid comprising dissolved chitosan/chitosan derivative and active ingredient.

The dry pharmaceutical compositions comprising an active ingredient selected from 5-ALA, a precursor of 5-ALA or a derivative of 5-ALA, and one or more polymers selected from cellulose ethers is preferably obtained as follows:
Cellulose ethers such as methylcellulose, ethylcellulose, hydroxyethylcellulose, hydroxymethlypropylcellulose, hydroxypropylcellulose or carboxymethylcellulose and the active ingredient are added to a solvent, preferably water or a mixture of water and an organic solvent, preferably alcohol such as ethanol. The so-obtained liquid is processed as described below.

A dry pharmaceutical composition according to the invention is preferably obtained from the above-described liquids by lyophilization, solvent evaporation or spray-coating as described hereinbefore.

In a preferred embodiment, said liquid is applied to an area for carrying a pharmaceutical composition comprised on an irradiation device for use in photodynamic therapy or photodynamic, e.g. filled in or applied to a reservoir on an irradiation device for use in photodynamic therapy of cancer, pre-cancerous conditions and non-cancerous conditions of the cervix as disclosed in Figs. 1-3 and 6-7 of WO 2010/078929. The device or a module thereof (e.g. just the reservoir) plus liquid is lyophilized or a deposit of the dry pharmaceutical composition in the reservoir is obtained by solvent evaporation. In another preferred embodiment, said liquid is applied to an area for carrying a pharmaceutical composition comprised on an irradiation device for use in PDT, e.g. to a device as disclosed in Figs. 1 to 7 of WO 2010/078929, by spray-coating.

The device is unpacked by a physician or nurse, inserted into the vagina and placed at the desired site cervix) where it stays during disintegration/dissolution of the dry pharmaceutical composition, incubation (i.e. build-up of porphyrins) and photodynamic treatment. If the device consists of modules, the module containing the dry pharmaceutical composition (e.g. the area for carrying a pharmaceutical composition such as a reservoir) is unpacked and combined with the other modules of the device to result in a fully working device. A timer may be part of said preferred device which, after being activated before the device is inserted, ensures that irradiation starts after the desired disintegration/dissolution/incubation period and continues for a determined light treatment period and diagnostic procedure, respectively. In a more preferred embodiment, the PDT device is disposable and adapted to be removed by the patient without the need to see his/her physician again.

After incubation, the site to be treated is exposed to light to achieve the desired photoactivation and photodynamic treatment or diagnosis. The length of time period between administration and exposure to light (disintegration/dissolution/incubation time) will depend on the nature of the active ingredient and the nature of the dry pharmaceutical composition. Generally, it is necessary that the active ingredient within said pharmaceutical composition is sufficiently released to be taken up by the cells of the tissue to be treated, converted into a photosensitiser and achieves an effective tissue concentration at the site of treatment prior to photoactivation. For PDT, the incubation time is about 30 min to 10 hours, preferably 1 hour to 7 hours, e.g. 3 hours to 5 hours.

The irradiation will generally be applied for a short time with a high light intensity, i.e. a high fluence rate or for a longer time with a low light intensity, i.e. low fluence rate. The latter is preferred for a PDT procedure wherein the dry pharmaceutical composition is comprised in a device as described in WO 2010/078929. With such devices, the light treatment can be carried out at a low fluence rate over a longer time period, e.g. at a fluence rate of 1 - 10 mW/cm² over a time period of several hours. This is beneficial both in terms of reduced discomfort to the patient and in the efficacy of the treatment.

The wavelength of light used for irradiation may be selected to achieve an efficacious photodynamic effect. Light having wavelengths of between 300-800 nm, for example, the range 400-700 nm has been found to be particularly effective. For PDT, it can be particularly important to include the wavelengths 630 and 690 nm. Red light (600 - 670 nm) is particularly preferred since light at this wavelength is known to penetrate well into tissue. Therefore the irradiation device preferably emits, in use, light having wavelengths of between 630-690 nm but more preferably light of a particular wavelength, e.g. about 630 nm.

For PDT, a single irradiation may be used or alternatively light may be split and delivered in a number of fractions, e.g. a few to several minutes between irradiations. Multiple irradiations may also be applied but are not preferred. Treatment of the patient is preferably carried out with a single dose of the dry pharmaceutical composition. However, if treatment is not complete, it can be repeated.

The disclosed dry pharmaceutical compositions, irradiation devices containing such compositions and methods for photodynamic therapy may be combined with other therapeutic procedures, for example administration of other therapeutic drugs. These therapeutic drugs might be administered to the patient prior to, together or subsequent to the dry pharmaceutical compositions. Other routes of administration may be oral, intravascular or dermal. Typical such drugs include hormones, antibacterial agents, antifungal agents, antiviral agents, anticancer agents or a combination of such drugs.

Also disclosed is a method of photodynamic treatment of cancer, pre-cancerous conditions and non-cancerous conditions of the cervix the said method comprising the steps of:
a) placing at a site of treatment on the cervix of a human or non-human animal subject an irradiation device according to the invention.
b) waiting for a time period necessary for the active ingredient within said dry pharmaceutical composition to be converted into a photosensitiser and achieve an effective therapeutic tissue concentration at the desired site; and
c) photoactivating the photosensitiser by exposing it to light from said device.

The invention is illustrated by the following non-limiting examples:

### Examples:

5-ALA hexyl ester hydrochloride (HAL HCl) was prepared as described in WO 96/28412; 5-ALA benzyl ester hydrochloride (BAL HCl) was prepared as described in WO 02/10120. 5-ALA methyl nitrate (MAL nitrate) was prepared from 5-ALA methyl hydrochloride (prepared as described in WO 96/28412) by the silver salt method described in WO 2005/092838. 5-ALA hexyl ester mesylate salt (HAL Mes) is prepared from 5-ALA hexyl ester hydrochloride (prepared as described in WO 96/28412) by the silver salt method described in WO 2005/092838.

### Example 1

Flexible collodion is prepared by dissolving 4 parts by weight of nitrocellulose in 75 parts by volume of diethyl ether and 25 parts by volume of ethanol. 2% by weight of camphor and 3% by weight of castor oil (plasticizers) are added and the mixture is stirred until a uniform pale yellow, syrupy liquid is obtained. 500 mg 5-ALA hexyl ester hydrochloride salt (HAL HCl) is added to 10 g of the flexible collodion and the mixture is stirred until a uniform liquid is obtained.

The reservoir of the devices according to Figs. 1-3 and 6-7 of WO 2010/078929 is filled with 2 g of the above-obtained liquid. The liquid is uniformly distributed in the reservoir and dried by solvent evaporation to form a film.

### Example 2 (not within the scope of the invention)

A liquid is prepared by dissolving 0.75 g methylcellulose and 0.08 g PEG 400 in 8.67 g of distilled water under stirring. 500 mg HAL HCl is added and the mixture is stirred until a uniform liquid is obtained.

A device according to Fig. 5 of WO 2010/078929 is spray-coated with 2 g of the above liquid. The liquid dries on the surface of the device (treatment surface) to form a film.

### Example 3 (not within the scope of the invention)

A liquid is prepared by dissolving 0.75 g methylcellulose and 0.16 g PEG 400 in 8.15 g of distilled water under stirring. 500 mg 5-ALA hexyl ester mesylate salt (HAL Mes) is added and the mixture is stirred until a uniform liquid is obtained.

A device according to Fig. 5 of WO 2010/078929 is spray-coated with 2 g of the above liquid. The liquid dries on the surface of the device (treatment surface) to form a film.

### Example 4

A liquid is prepared by mixing 500 mg HAL HCl, 4.5 g chitosan glutamate and 5 g distilled water. The mixture is stirred until a uniform dissolution is obtained.

The reservoir part of the devices according to Figs. 1-3 and 6-7 of WO 2010/078929 is filled with 2 g of the above-obtained liquid. The reservoir part is frozen in liquid nitrogen and lyophilized. A dry pharmaceutical composition comprising 10% HAL HCl and 90% chitosan glutamate by weight of the dry pharmaceutical composition is obtained in the reservoir. The reservoir is connected to the remainder of the device. The device comprising the dry pharmaceutical composition can be used for the photodynamic treatment of cancerous, pre-cancerous or non-cancerous conditions of the cervix.

### Example 5

A liquid is prepared by mixing 500 mg HAL Mes, 4.5 g chitosan lactate and 5 g distilled water. The mixture is stirred until a uniform dissolution is obtained.

The reservoir part of the devices according to Figs. 1-3 and 6-7 of WO 2010/078929 is filled with 2 g of the above-obtained liquid. The reservoir part is frozen in liquid nitrogen and lyophilized. A dry pharmaceutical composition comprising 10% HAL Mes and 90% chitosan lactate by weight of the dry pharmaceutical composition is obtained in the reservoir. The reservoir is connected to the remainder of the device. The device plus drug can be used for the photodynamic treatment of cancerous, pre-cancerous or non-cancerous conditions of the cervix.

### Example 6 (not within the scope of the invention)

An aqueous liquid is prepared containing by weight 5.5% Eudragit RL30D (as 30% w/w dispersion), 1.1% Citroflex 2 (plasticizer) and 5% HAL HCl.

A device according to Fig. 5 of WO 2010/078929 is spray-coated with 2 g of the above liquid. The liquid dries on the surface of the device (treatment surface) to form a film.

### Example 7 (not within the scope of the invention)

9.5 g of an aqueous liquid is prepared containing by weight 20% pullulan and 1% sodium alginate. The liquid is left to stand overnight. 0.5 g HAL HCl is added and the liquid is stirred vigorously until a uniform, relatively low viscous liquid is obtained.

A device according to Fig. 5 of WO 2010/078929 is spray-coated with 2 g of the above liquid. The liquid dries on the surface of the device (treatment surface) to form a film.

### Example 8 (not within the scope of the invention)

9.5 g of an aqueous liquid is prepared containing by weight 20% pullulan and 1% sodium alginate. The liquid is left to stand overnight. The pH of the liquid is adjusted to 3.5 using diluted hydrochloric acid. 0.5 g HAL HCl is added and the liquid is stirred vigorously until a uniform, relatively high viscous liquid is obtained. A device according to Fig. 5 of WO 2010/078929 is dip-coated with 2 g of the above liquid. The liquid dries on the surface of the device (treatment surface) to form a film.

### Example 9

9.5 g of an aqueous liquid is prepared containing 10% by weight chitosan and 1% (vol/vol) acetic acid 0.5 g HAL HCl is added and the mixture is stirred until uniform.

The reservoir part of the devices according to Figs. 1-3 and 6-7 of WO 2010/078929 is filled with 2 g of the above-obtained liquid. The reservoir part is frozen in liquid nitrogen and lyophilized. A dry pharmaceutical composition comprising 34.5% HAL HCl and 65.5% chitosan lactate is obtained in the reservoir. The reservoir is connected to the remainder of the device. The device plus drug can be used for the photodynamic treatment of cancerous, pre-cancerous or non-cancerous conditions of the cervix.

In the following experiments, a round-bottom glass flask was used to copy the concave/frustoconical surface shape of the reservoir of a device according to Figs. 1-3 and 6-7 of WO 2010/078929. While such a round-bottom glass flask is suitable to model the shape of the reservoir it has however different surface properties due to the different materials, i.e. glass versus. a resilient material commonly used in medical devices such as rubber, latex, silicone or other polymers and co-polymers.

### Example 10

A liquid was prepared by dissolving 100 mg HAL HCl and 10 mg methylcellulose 1500 (Apotekproduksjon AS, Oslo, Norway) in a mixture of water (10 ml) and 96% ethanol (10 ml) in a round-bottom glass flask. After freeze-drying, a mechanically stable homogeneous film was obtained.

### Example 11

A liquid was prepared by dissolving 100 mg HAL HCl and 25 mg methylcellulose 1500 (Apotekproduksjon AS, Oslo, Norway) in a mixture of water (10 ml) and 96% ethanol (10 ml) in a round-bottom glass flask. After freeze-drying, a homogeneous film was obtained which was not fully attached to the glass surface.

### Example 12

A liquid was prepared by dissolving 100 mg HAL HCl and 50 mg methylcellulose 1500 (Apotekproduksjon AS, Oslo, Norway) in a mixture of water (10 ml) and 96% ethanol (10 ml) in a round-bottom glass flask. After freeze-drying, a homogeneous film was obtained which was not fully attached to the glass surface.

### Example 13

A liquid was prepared by dissolving 100 mg HAL HCl and 10 mg ethylcellulose (Sigma Aldrich #200646) in water (10 ml) in a round-bottom glass flask. After freeze-drying, a fluffy cake was obtained.

### Example 14

A liquid was prepared by dissolving 100 mg HAL HCl and 10 mg hydroxyethylcellulose 250HX Pharm (Fagron GmbH & Co KG, Barsbüttel, Germany) in water (10 ml) in a round-bottom glass flask. After freeze-drying, a somewhat inhomogeneous film was obtained which was not fully attached to the glass surface.

### Example 15

A liquid was prepared by dissolving 100 mg HAL HCl and 10 mg chitosan (Sigma Aldrich # 448877) in water (10 ml) in a round-bottom glass flask. After freeze-drying, a somewhat inhomogeneous, net-like film was obtained which was not fully attached to the glass surface.

### Example 16

A liquid was prepared by dissolving 100 mg HAL HCl and 10 mg carboxymethylcellulose sodium salt (Sigma Aldrich # C5678) in water (10 ml) in a round-bottom glass flask. After freeze-drying, a somewhat inhomogeneous, net-like film was obtained which was not fully attached to the glass surface.

### Example 17

A liquid was prepared by dissolving 100 mg 5-ALA benzyl ester hydrochloride salt (BAL HCl) and methylcellulose 1500 (Apotekproduksjon AS, Oslo, Norway) in water (10 ml) in a round-bottom glass flask. The amount of methylcellulose was 10 mg, 25 mg and 50 mg, respectively. After freeze-drying, somewhat inhomogeneous, net-like films were obtained which were not fully attached to the glass surface.

### Example 18

A liquid was prepared by dissolving 100 mg HAL HCl, 25 mg methylcellulose 1500 (Apotekproduksjon AS, Oslo, Norway) and 3 mg triethyl citrate (Merck Chemicals, Northern Europe) in water (10 ml) in a round-bottom glass flask. After freeze-drying, a relatively homogeneous, mechanically stable soft film was obtained.

### Example 19

A liquid was prepared by dissolving 100 mg HAL HCl and 25 mg methylcellulose 1500 (Apotekproduksjon AS, Oslo, Norway) in water (10 ml) in a round-bottom glass flask. After freeze-drying, a relatively homogeneous, mechanically stable soft film was obtained.

### Example 20

A liquid was prepared by dissolving 100 mg BAL HCl, 25 mg hydroxymethylpropylcellulose and 3 mg triethyl citrate (Merck Chemicals, Northern Europe) in water (10 ml) in a round-bottom glass flask. After freeze-drying, a relatively homogeneous, mechanically stable soft film was obtained.

### Example 21

A liquid was prepared by dissolving 100 mg BAL HCl and 25 mg hydroxymethylpropylcellulose in water (10 ml) in a round-bottom glass flask. After freeze-drying, a relatively homogeneous, mechanically stable soft film was obtained.

### Example 22

A liquid was prepared by dissolving 100 mg HAL HCl, 200 mg Kollicoat IR White, a polyvinyl alcohol-polyethylene glycol graft polymer containing titanium dioxide pigments (BASF, Ludwigshafen, Germany) and 50 mg Kollicoat IR Yellow, a polyvinyl alcohol-polyethylene glycol graft polymer containing titanium dioxide and iron oxide pigments (BASF, Ludwigshafen, Germany) in water (10 ml) in a round-bottom glass flask. After freeze-drying, a relatively homogeneous, mechanically stable yellow film was obtained.

In the following experiments, an irradiation device according to Fig. 6 of WO 2010/078929 was used. The reservoir of said device (63 in Fig. 6) is made of silicone.

### Example 23

A liquid was prepared by dissolving 100 mg HAL HCl, 25 mg methylcellulose 1500 (Apotekproduksjon AS, Oslo, Norway) and 3 mg triethyl citrate (Merck Chemicals, Northern Europe) in 2 ml water. The reservoir of the irradiation device was coated with the solution and the device was freeze-dried. After freeze-drying, a homogeneous soft mechanically stable film was obtained.

### Example 24

A liquid was prepared by dissolving 100 mg HAL HCl and 25 mg methylcellulose 1500 (Apotekproduksjon AS, Oslo, Norway) in 2 ml water. The reservoir of the irradiation device was coated with the solution using a brush and the device was freeze-dried. After freeze-drying, a homogeneous, soft, slightly brittle film was obtained which was less mechanically stable than the film obtained in Example 23.

### Example 25

A liquid was prepared by dissolving 100 mg BAL HCl, 25 mg methylcellulose 1500 (Apotekproduksjon AS, Oslo, Norway) and 3 mg triethyl citrate (Merck Chemicals, Northern Europe) in 2 ml water. The reservoir of the irradiation device was coated with the solution using a brush and the device was freeze-dried. After freeze-drying, a homogeneous, soft, slightly brittle film was obtained which was less mechanically stable than the film obtained in Example 23.

### Example 26

A liquid was prepared by dissolving 100 mg BAL HCl and 25 mg methylcellulose 1500 (Apotekproduksjon AS, Oslo, Norway) in 2 ml water. The reservoir of the irradiation device was coated with the solution using a brush and the device was freeze-dried. After freeze-drying, a homogeneous, soft, slightly brittle film was obtained which was less mechanically stable than the film obtained in Example 23.

### Example 27

A liquid was prepared by dissolving 100 mg HAL HCl and 25 mg hydroxymethylpropylcellulose in 2 ml water. The reservoir of the irradiation device was coated with the solution using a brush and the device was freeze-dried. After freeze-drying, a homogeneous, soft, slightly brittle film was obtained which was less mechanically stable than the film obtained in Example 23.

### Example 28

A liquid was prepared by dissolving 100 mg BAL HCl and 25 mg hydroxymethylpropylcellulose in 2 ml water. The reservoir of the irradiation device was coated with the solution using a brush and the device was freeze-dried. After freeze-drying, a mechanically stable cake was obtained.

### Example 29

A liquid was prepared by dissolving 100 mg BAL HCl, 25 mg hydroxymethylpropylcellulose and 3 mg triethyl citrate (Merck Chemicals, Northern Europe) in 2 ml water. The reservoir of the irradiation device was coated with the solution using a brush and the device was freeze-dried. After freeze-drying, a mechanically stable cake was obtained.

In the following experiments, the irradiation device used in Examples 23 to 29 was pretreated to improve the wetting properties of the silicone material towards aqueous solutions: 12N hydrochloric acid (aq) was filled into the reservoir of the device and left there for 30 min. The reservoir was emptied and several times washed with water. No visual change in the silicone material could be observed.

### Example 30

A liquid was prepared by dissolving 100 mg HAL HCl and 10 mg methylcellulose 1500 (Apotekproduksjon AS, Oslo, Norway) in 2 ml water. The device was kept on dry ice for 15 min before coating. The reservoir of the irradiation device was coated with the solution using a brush and the device was freeze-dried. After freeze-drying, a homogeneous, medium soft, mechanically stable film was obtained.

### Example 31

A liquid was prepared by dissolving 100 mg BAL HCl and 10 mg methylcellulose 1500 (Apotekproduksjon AS, Oslo, Norway) in 2 ml water. The device was kept on dry ice for 15 min before coating. The reservoir of the irradiation device was coated with the solution using a brush and the device was freeze-dried. After freeze-drying, a homogeneous, medium soft, mechanically stable film was obtained.

### Example 32

A liquid was prepared by dissolving 100 mg HAL HCl and 12.5 mg hydroxypropylmethylcellulose in 5 ml water. The reservoir of the irradiation device was coated with the solution using a brush and the device was freeze-dried. After freeze-drying, a fluffy cake was obtained.

### Example 33

A liquid was prepared by dissolving 100 mg HAL HCl and 10 mg hydroxypropylmethylcellulose in 2 ml water. The device was kept on dry ice for 15 min before coating. The reservoir of the irradiation device was coated with the solution using a brush and the device was freeze-dried. After freeze-drying, a homogeneous, medium soft, mechanically stable film was obtained.

### Example 34

A liquid was prepared by dissolving 100 mg BAL HCl and 10 mg hydroxypropylmethylcellulose in 2 ml water. The device was kept on dry ice for 15 min before coating. The reservoir of the irradiation device was coated with the solution using a brush and the device was freeze-dried. After freeze-drying, a homogeneous, medium soft, mechanically stable film was obtained.

### Example 35

A liquid was prepared by dissolving the following compounds in 2 ml water:
35a: 20 mg HAL HCl and 40 mg hydroxypropylmethylcellulose
35b: 20 mg HAL HCl, 40 mg hydroxypropylmethylcellulose and 5 mg PEG 600
35c: 20 mg HAL HCl, 40 mg hydroxypropylmethylcellulose and 15 mg PEG 600
35d: 20 mg HAL HCl, 40 mg hydroxypropylmethylcellulose and 60 mg PEG 600

The device was kept on dry ice for 15 min before coating. The reservoirs of the irradiation devices were coated with the solutions using a brush and the devices were freeze-dried.

After freeze-drying, the following was obtained:
35a: Medium soft, somewhat inhomogeneous film. Mechanically stable.
35b: Relatively hard, homogeneous film. Mechanically stable.
35c: Relatively hard, homogeneous film. Mechanically stable.
35d: Hard, homogeneous film. Mechanically stable.

### Example 36

A liquid was prepared by dissolving the following compounds in 2 ml water:
36a: 20 mg methyl 5-ALA nitrate (MAL nitrate), 40 mg hydroxypropylmethylcellulose and 30 mg PEG 600
36b: 20 mg MAL nitrate, 40 mg hydroxypropylmethylcellulose and 14 mg PEG 200

The device was kept on dry ice for 15 min before coating. The reservoirs of the irradiation devices were coated with the solutions using a brush and the devices were freeze-dried. After freeze-drying, the following was obtained:
36a: Hard homogeneous film. Mechanically stable.
36b: Hard homogeneous film. Mechanically stable.

### Example 37

A liquid was prepared by dissolving 100 mg HAL HCl, 40 mg hydroxypropylmethylcellulose and 30 mg PEG 600 in 4 ml water. The device was kept on dry ice for 15 min before coating. The reservoir of the irradiation device was coated with the solution using a brush and the device was freeze-dried. After freeze-drying, a homogeneous, soft, mechanically stable film was obtained.

### Example 38

A liquid was prepared by dissolving 100 mg HAL HCl, 40 mg hydroxypropylmethylcellulose and 30 mg PEG 950-1050 in 2 ml water. The device was kept on dry ice for 15 min before coating. The reservoir of the irradiation device was coated with the solution using a brush and the device was freeze-dried. After freeze-drying, a homogeneous, hard, mechanically stable film was obtained.

### Example 39

The stability of certain dry compositions according to the invention was assessed as follows: after preparation of the dry composition in round-bottom glass flasks, the compositions were left within the glass flasks for up to 4 weeks at 37 °C. 5 ml water was added to the dry composition and the composition was dissolved. A 1 ml sample was withdrawn and filtered through a 0.45 µm syringe filter. 25 µl of the filtered sample was analyzed by HPLC (Agilent 1100, pump rate 1 ml/min) using a 4.4 x 250 mm ZORBAX extend C18 column (Agilent) and methanol/water (70:30 v/v) as eluent. UV detection was carried out at 210 nm. To determine % of ALA ester in the sample, the peak areas were calculated against a standard. The following dry compositions all showed a very good stability, i.e. the ALA-ester remained stable: Examples 20 and 21 after 25 and 27 days and Examples 18 and 19 after 8 days.

### Example 40

The release of ALA-ester from certain dry compositions upon contact of the reservoir of the irradiation device with water was assessed as follows:
The reservoir of the irradiation device was filled with 5 ml water (37°C) and the device was gently agitated for about 30 minutes. The liquid was removed from the device and filtered through a 0.45 µm syringe filter. The filtered liquid was analyzed as described in Example 39. A release of 100% corresponds to complete release of the ALA-ester. The following abbreviations are used: MC: methylcellulose, HPMC: hydroxypropylmethylcellulose, TC: triethyl citrate.

| **Example #** | **ALA-ester** | **Polymer** | **Plasticizer** | **Release [%]** |
|---|---|---|---|---|
| 23 | HAL HCl 100 mg | MC 25 mg | TC 3 mg | 97 |
| 24 | HAL HCl 100 mg | MC 25 mg | none | 72 |
| 25 | BAL HCl 100 mg | MC 25 mg | TC 3 mg | 64 |
| 26 | BAL HCl 100 mg | MC 25 mg | none | 72 |
| 27 | HAL HCl 100 mg | HPMC 25 mg | none | 85 |
| 28 | BAL HCl 100 mg | HPMC 25 mg | none | 56 |
| 29 | BAL HCl 100 mg | HPMC 25 mg | TC 3 mg | 83 |
| 32 | HAL HCl 100 mg | HPMC 12.5 mg | none | 70 |

All dry compositions released the active ingredient (ALA-ester) in a sufficient rate within 30 min of being contacted with water.

## Claims

1. An irradiation device for use in photodynamic therapy of cancer, pre-cancerous conditions and non-cancerous conditions of the cervix which comprises, in an area for carrying a pharmaceutical composition, a dry pharmaceutical composition,
wherein said device is adapted for full and secure insertion into the vagina and is independently operational while located in the vagina, and wherein the device comprises a housing adapted to be fully inserted and secured in the vagina, the housing enclosing a LED lamp system and a power source for powering the LED lamp system,
and wherein said housing comprises an upper housing portion which is approximately frustoconical in shape and whose front end forms a treatment surface which is shaped so as to cover, in use, the portio and the opening of the cervix and which is capable of providing irradiation to said areas, the treatment surface forming a reservoir for housing said dry pharmaceutical composition,
wherein said dry pharmaceutical composition comprises:
a) an active ingredient selected from 5-ALA, a precursor of 5-ALA or a derivative of 5-ALA, and pharmaceutically acceptable salts thereof;
b) one or more polymers which have good film-forming properties and/or good gel-forming properties; and
c) optionally other pharmaceutically acceptable excipients.

2. An irradiation device as claimed in claim 1, wherein the dry pharmaceutical composition is in the form of a powder, a cake, or a film.

3. An irradiation device as claimed in claim 2, wherein the dry pharmaceutical composition is in the form of a film.

4. An irradiation device as claimed in any one of the preceding claims, wherein the dry pharmaceutical composition is obtained by a film coating process, by solvent evaporation or by lyophilization.

5. An irradiation device as claimed in claim 4, wherein the film coating process is dip-coating or spray-coating.

6. An irradiation device as claimed in any one of the preceding claims, wherein said dry pharmaceutical composition comprises an active ingredient selected from a derivative of 5-ALA or a pharmaceutically acceptable salt thereof.

7. An irradiation device as claimed in claim 6, wherein said derivative of 5-ALA is a 5-ALA ester.

8. An irradiation device as claimed in claim 7, wherein said dry pharmaceutical composition comprises a 5-ALA ester of formula I or a pharmaceutically acceptable salt thereof:
R²₂N-CH₂COCH₂-CH₂CO-OR¹ (I)
wherein
R¹ represents a substituted or unsubstituted alkyl group; and
R² each independently represents a hydrogen atom or a group R¹.

9. An irradiation device as claimed in any one of the preceding claims, wherein said one or more polymers are selected from cellulose ethers, gellan gum, chitosan, chitosan derivatives, pullulan, alginates, hyaluronic acid, hyaluronic acid derivatives and carrageenan.

10. An irradiation device as claimed in claim 9, wherein said one or more polymers are selected from chitosan, chitosan derivatives and cellulose ethers.

11. An irradiation device as claimed in any one of the preceding claims, wherein the dry pharmaceutical composition consists of the active ingredient and one or more polymers, preferably one or more polymers selected from cellulose ethers, gellan gum, chitosan, chitosan derivatives, pullulan, alginates, hyaluronic acid, hyaluronic acid derivatives and carrageenan, more preferably one or more polymers selected from chitosan, chitosan derivatives and cellulose ethers.

12. An irradiation device as claimed in any one of claims 1 to 4 and 6 to 11, wherein the dry pharmaceutical composition is obtained by lyophilization.

13. An irradiation device as claimed in claim 12 wherein the dry pharmaceutical composition is obtained by lyophilization of a liquid which is prepared by dissolving or suspending the active ingredient and one or more polymers which have good film-forming properties and/or good gel-forming properties and optionally other pharmaceutically acceptable excipients in a suitable solvent.

14. An irradiation device as claimed in claim 13, wherein said suitable solvent is water or a mixture of solvents, preferably water and alcohols.

15. An irradiation device as claimed in any one of claims 1 to 10 and 12 to 14, wherein the dry pharmaceutical composition further comprises one or more other pharmaceutically acceptable excipients, preferably one or more other pharmaceutically acceptable excipients selected from plasticizers, coloring agents, thickening agents, disintegrants, mucoadhesive agents, surface penetration enhancing agents and/or chelating agents.

## Patentansprüche

1. Bestrahlungsvorrichtung zur Verwendung bei einer fotodynamischen Therapie von Krebs, präkanzerösen Zuständen und nicht-kanzerösen Zuständen der Zervix, welche in einer Fläche zum Tragen einer pharmazeutischen Zusammensetzung eine trockene pharmazeutische Zusammensetzung umfasst,
wobei die Vorrichtung zum vollständigen und sicheren Einschub in die Vagina angepasst ist und unabhängig einsatzfähig ist, während sie in der Vagina angeordnet wird, und wobei die Vorrichtung ein Gehäuse, angepasst, um vollständig in die Vagina eingeschoben und befestigt zu werden, umfasst, wobei das Gehäuse ein LED-Lampensystem und eine Stromquelle zum Antreiben des LED-Lampensystems umschließt,
und wobei das Gehäuse einen oberen Gehäuseteil umfasst, welcher in der Form ungefähr kegelstumpfförmig ist, und dessen vorderes Ende eine Behandlungsoberfläche bildet, welche so geformt ist, um bei Gebrauch die Portio und die Öffnung der Zervix abzudecken und welche Bestrahlung der Flächen bereitstellen kann, wobei die Behandlungsoberfläche ein Reservoir für das Gehäuse der trockenen pharmazeutischen Zusammensetzung bildet,
wobei die trockene pharmazeutische Zusammensetzung umfasst:
a) einen Wirkstoff, ausgewählt aus 5-ALA, einer Vorstufe von 5-ALA oder einem Derivat von 5-ALA und pharmazeutisch verträglichen Salzen davon;
b) ein oder mehrere Polymere, welche gute Film-bildende Eigenschaften und/oder gute Gel-bildende Eigenschaften aufweisen; und
c) gegebenenfalls weitere pharmazeutisch verträgliche Exzipienten.

2. Bestrahlungsvorrichtung nach Anspruch 1, wobei die trockene pharmazeutische Zusammensetzung in Form von einem Pulver, einem Kuchen oder einem Film vorliegt.

3. Bestrahlungsvorrichtung nach Anspruch 2, wobei die trockene pharmazeutische Zusammensetzung in Form von einem Film vorliegt.

4. Bestrahlungsvorrichtung nach einem der vorstehenden Ansprüche, wobei die trockene pharmazeutische Zusammensetzung durch ein Filmbeschichtungsverfahren, durch Lösungsmittelverdampfung oder durch Lyophilisierung erhalten wird.

5. Bestrahlungsvorrichtung nach Anspruch 4, wobei das Filmbeschichtungsverfahren Tauchbeschichtung oder Sprühbeschichtung ist.

6. Bestrahlungsvorrichtung nach einem der vorstehenden Ansprüche, wobei die trockene pharmazeutische Zusammensetzung einen Wirkstoff, ausgewählt aus einem Derivat von 5-ALA oder einem pharmazeutisch verträglichen Salz davon, umfasst.

7. Bestrahlungsvorrichtung nach Anspruch 6, wobei das Derivat von 5-ALA ein 5-ALA-Ester ist.

8. Bestrahlungsvorrichtung nach Anspruch 7, wobei die trockene pharmazeutische Zusammensetzung einen 5-ALA-Ester der Formel I oder ein pharmazeutisch verträgliches Salz davon umfasst:
R²₂N-CH₂COCH₂-CH₂CO-OR¹ (I)
worin
R¹ eine substituierte oder unsubstituierte Alkylgruppe wiedergibt; und
R² jeweils unabhängig ein Wasserstoffatom oder eine Gruppe R¹ wiedergibt.

9. Bestrahlungsvorrichtung nach einem der vorstehenden Ansprüche, wobei die einen oder mehreren Polymere aus Celluloseethern, Gellangummi, Chitosan, Chitosan-Derivaten, Pullulan, Alginaten, Hyaluronsäure, Hyaluronsäure-Derivaten und Carrageenan ausgewählt sind.

10. Bestrahlungsvorrichtung nach Anspruch 9, wobei das eine oder mehrere Polymere aus Chitosan, Chitosan-Derivaten und Celluloseethern ausgewählt sind.

11. Bestrahlungsvorrichtung nach einem der vorstehenden Ansprüche, wobei die trockene pharmazeutische Zusammensetzung aus dem Wirkstoff und einem oder mehreren Polymeren, vorzugsweise einem oder mehreren Polymeren, ausgewählt aus Celluloseethern, Gellangummi, Chitosan, Chitosan-Derivaten, Pullulan, Alginaten, Hyaluronsäure, Hyaluronsäure-Derivaten und Carrageenan, bevorzugter einem oder mehreren Polymeren, ausgewählt aus Chitosan, Chitosan-Derivaten und Celluloseethern, besteht.

12. Bestrahlungsvorrichtung nach einem der Ansprüche 1 bis 4 und 6 bis 11, wobei die trockene pharmazeutische Zusammensetzung durch Lyophilisierung erhalten wird.

13. Bestrahlungsvorrichtung nach Anspruch 12, wobei die trockene pharmazeutische Zusammensetzung durch Lyophilisierung einer Flüssigkeit erhalten wird, welche durch Auflösen oder Suspendieren des Wirkstoffs und eines oder mehrerer Polymere, welche gute Film-bildende Eigenschaften und/oder gute Gel-bildende Eigenschaften aufweisen, und gegebenenfalls anderer pharmazeutisch verträglicher Exzipienten in einem geeigneten Lösungsmittel hergestellt wird.

14. Bestrahlungsvorrichtung nach Anspruch 13, wobei das geeignete Lösungsmittel Wasser oder ein Gemisch von Lösungsmitteln, vorzugsweise Wasser und Alkohole, ist.

15. Bestrahlungsvorrichtung nach einem der Ansprüche 1 bis 10 und 12 bis 14, wobei die trockene pharmazeutische Zusammensetzung weiterhin ein oder mehrere weitere pharmazeutisch verträgliche Exzipienten, vorzugsweise ein oder mehrere weitere pharmazeutisch verträgliche Exzipienten, ausgewählt aus Weichmachern, Färbemitteln, Verdickungsmitteln, Zerfallsmitteln, mukoadhäsiven Mitteln, Oberflächen-Penetration verstärkenden Mitteln und/oder chelatisierenden Mitteln, umfasst.

## Revendications

1. Dispositif d'irradiation destiné à être utilisé dans la thérapie photodynamique du cancer, des états pré-cancéreuses et des états non-cancéreux du col de l'utérus, qui comprend, dans une zone servant de support à une composition pharmaceutique, une composition pharmaceutique sèche,
dans lequel ledit dispositif est adapté pour une insertion complète et sûre dans le vagin et est opérationnel de manière indépendante lorsque logé dans le vagin, et dans lequel le dispositif comprend un logement adapté pour être complètement inséré et fixé dans le vagin, le boîtier renfermant un système de lampe LED et une source d'alimentation pour alimenter le système de lampe LED,
et dans lequel ledit logement comprend une partie de boîtier supérieure qui a une forme approximativement tronconique et dont l'extrémité avant forme une surface de traitement qui est formée de façon à recouvrir, en cours d'utilisation, la portion inférieure et l'ouverture du col de l'utérus et qui est capable de fournir une irradiation auxdites zones, la surface de traitement formant un réservoir pour loger ladite composition pharmaceutique sèche,
dans lequel ladite composition pharmaceutique sèche comprend :
a) un ingrédient actif choisi parmi le 5-ALA, un précurseur du 5-ALA ou un dérivé du 5-ALA et leurs sels pharmaceutiquement acceptables ;
b) un ou plusieurs polymères qui ont de bonnes propriétés filmogènes et/ou de bonnes propriétés de formation de gel ; et
c) en option d'autres excipients pharmaceutiquement acceptables.

2. Dispositif d'irradiation selon la revendication 1, dans lequel la composition pharmaceutique sèche se présente sous la forme d'une poudre, d'un gâteau ou d'un film.

3. Dispositif d'irradiation selon la revendication 2, **caractérisé en ce que** la composition pharmaceutique sèche se présente sous la forme d'un film.

4. Dispositif d'irradiation selon l'une quelconque des revendications précédentes, dans lequel la composition pharmaceutique sèche est obtenue par un procédé de revêtement par film, par évaporation de solvant ou par lyophilisation.

5. Dispositif d'irradiation selon la revendication 4, dans lequel le procédé de revêtement par film est un revêtement par immersion ou par pulvérisation.

6. Dispositif d'irradiation selon l'une quelconque des revendications précédentes, dans lequel ladite composition pharmaceutique sèche comprend un ingrédient actif choisi parmi un dérivé de 5-ALA ou un de ses sels pharmaceutiquement acceptables.

7. Dispositif d'irradiation selon la revendication 6, dans lequel ledit dérivé de 5-ALA est un ester de 5-ALA.

8. Dispositif d'irradiation selon la revendication 7, dans lequel ladite composition pharmaceutique sèche comprend un ester de 5-ALA de formule I ou un de ses sels pharmaceutiquement acceptables :
R²₂N-CH₂COCH₂-CH₂CO-OR¹ (I)
dans laquelle
R¹ représente un groupe alkyle substitué ou non substitué; et
R² représentent chacun indépendamment un atome d'hydrogène ou un groupe R¹.

9. Dispositif d'irradiation selon l'une quelconque des revendications précédentes, dans lequel ledit ou lesdits polymères sont choisis parmi les éthers de cellulose, la gomme gellane, le chitosan, les dérivés de chitosan, le pullulane, les alginates, l'acide hyaluronique, les dérivés d'acide hyaluronique et le carraghénane.

10. Dispositif d'irradiation selon la revendication 9, dans lequel ledit ou lesdits polymères sont choisis parmi le chitosan, les dérivés de chitosan et les éthers de cellulose.

11. Dispositif d'irradiation selon l'une quelconque des revendications précédentes, dans lequel la composition pharmaceutique sèche consiste en l'ingrédient actif et un ou plusieurs polymères, de préférence un ou plusieurs polymères choisis parmi les éthers de cellulose, la gomme de gellane, le chitosan, les dérivés de chitosan, le pullulane, les alginates, l'acide hyaluronique, les dérivés d'acide hyaluronique et le carraghénane, de préférence encore un ou plusieurs polymères choisis parmi le chitosan, les dérivés de chitosan et les éthers de cellulose.

12. Dispositif d'irradiation selon l'une quelconque des revendications 1 à 4 et 6 à 11, dans lequel la composition pharmaceutique sèche est obtenue par lyophilisation.

13. Dispositif d'irradiation selon la revendication 12, dans lequel la composition pharmaceutique sèche est obtenue par lyophilisation d'un liquide qui est préparé en dissolvant ou en suspendant l'ingrédient actif et un ou plusieurs polymères qui ont de bonnes propriétés filmogènes et/ou de bonnes propriétés de formation de gel et éventuellement d'autres excipients pharmaceutiquement acceptables dans un solvant approprié.

14. Dispositif d'irradiation selon la revendication 13, dans lequel ledit solvant approprié est l'eau ou un mélange de solvants, de préférence de l'eau et des alcools.

15. Dispositif d'irradiation selon l'une quelconque des revendications 1 à 10 et 12 à 14, dans lequel la composition pharmaceutique sèche comprend en outre un ou plusieurs autres excipients pharmaceutiquement acceptables, de préférence un ou plusieurs autres excipients pharmaceutiquement acceptables sélectionnés parmi les plastifiants, les agents colorants, les agents épaississants, les agents désagrégation, les agents muco-adhésifs, les agents améliorant la pénétration de surface et/ou les agents chélatants.
